# EUROPEAN PATENT SPECIFICATION

(11) **EP 3 610 836 B1**
(45) Date of publication and mention of the grant of the patent: **28.04.2021**
(21) Application number: 19189559.8
(22) Date of filing: 01.08.2019
(51) Int. Cl.: A61F 11/08

(54) **A SOUND DAMPING FILTER FOR A HEARING PROTECTOR PLACEABLE IN THE AUDITORY DUCT OF A PERSON, AS WELL AS A CORRESPONDING HEARING PROTECTOR**
SCHALLDÄMPFUNGSFILTER FÜR EINEN IM GEHÖRGANG EINER PERSON PLATZIERBAREN GEHÖRSCHUTZ SOWIE ENTSPRECHENDER GEHÖRSCHUTZ
FILTRE D'AMORTISSEMENT ACOUSTIQUE POUR DISPOSITIF DE PROTECTION AUDITIVE À INSTALLER DANS LE CONDUIT AUDITIF D'UNE PERSONNE, AINSI QU'UN DISPOSITIF DE PROTECTION AUDITIVE CORRESPONDANT

(30) Priority: 16.08.2018 NL 2021475
(43) Date of publication of application: 19.02.2020
(73) Proprietor: Elacin International B.V., 5107 RG Dongen (NL)
(72) Inventor: FLESKENS, Bas, 5107RG DONGEN (NL); VERHAVE, Jan Adrianus, 5107RG DONGEN (NL)
(74) Representative: Algemeen Octrooi- en Merkenbureau B.V.

(56) References cited:
- WO-A1-01/76520
- WO-A1-2011/078660
- WO-A1-2018/117821

## Description

The present disclosure is directed to a sound damping filter for a hearing protector placeable in the auditory duct of a person, comprising a sound damping canal with a passage extending from a sound inlet opening to a sound outlet opening.

Such a filter is known from practice and is utilized in a hearing protector placeable in the auditory duct of a person in order to damp the intensity of sound to be supplied to the eardrum of the person. By varying the cross section and the length of the sound damping canal, the level of damping can be influenced, and a sound damping filter can be manufactured which is tailored to a particular application.

The length of the auditory duct of a person results in a resonance frequency (1/4 wavelength), which is typically between 2.5 - 3.5 kHz. Sound within this frequency range is amplified by about 10 - 15dB due to the resonant cavity effect.

Also known are filters for hearing protectors where the passage of the damping canal is closed off by means of a membrane. Although a hearing protector with such a filter enables a damping level that remains substantially constant in magnitude over the audible frequency range, there are disadvantages with this type of hearing protector as well.

It is found that the resonance frequency, as described above, disappears to a certain extend whenever a hearing protector is placed in the auditory duct of a person. The auditory duct is normally open at one side, and closed with the eardrum at the other side thereof. The auditory duct is no longer fully open at one side, once the hearing protector is placed therein.

One of the downsides of the known hearing protectors is that they do not handle the resonance frequency aspect, as described above, adequately

WO 2018/117821 discloses a sound filter for a hearing protector.

It is therefore an object of the present disclosure to provide for a sound damping filter which offers a resonance frequency which is, or may be, tuned to a desired frequency range and therefore does not possess the above mentioned disadvantage.

It is a further object to provide for a hearing protector having the found damping filter.

In a first object, there is provided a sound damping filter for a hearing protector placeable in the auditory duct of a person, said sound damping filter comprising a sound damping canal, wherein said sound damping canal comprises a first tube part connected to a second tube part, wherein said first tube part provides for a sound inlet opening and wherein said second tube part provides for a sound outlet opening such that a sound passage extends in said sound damping canal from said sound inlet opening to said sound outlet opening, wherein said first tube part comprises at least one membrane providing a sound damping function, and wherein said sound inlet opening is larger than said sound outlet opening.

Typically, the second tube part forms a protrusion. That is, it is relatively short compared to the first tube part and the passage through the second tube part is substantially smaller compared to the passage through the first tube part.

It was the insight of the inventors that the introduction of the second tube part is advantageous as it accomplishes that the damping in reduced particular range may be provided. This could accomplish that the loss of the resonance frequency is compensated for in a desired frequency range.

It is noted that the tuning of the dimensions of the second tube part may be advantageous as it may accomplish that the damping is tuned to a particular frequency range, for example 2.5 - 3.5 kHz, provided a certain resonance frequency to be present inside the system. Adequate tuning of the dimensions of the second tube part allows for a tailored acoustic resistance which can displace any present resonance over the frequency range.

In an example, the first tube part and said second tube part are shaped cylindrically.

More specifically, said first tube part may be formed such that said sound inlet opening has a diameter of between 4mm - 6mm, preferably substantially 5mm, and said second tube part may be formed such that said sound outlet opening has a diameter of between 0,3mm - 1,0mm, preferably substantially 0,6mm.

It was found that the above described ranges for the sound inlet opening and the sound outlet opening are beneficial for the damping characteristic of the hearing protector.

Typically, it is desired to obtain a relatively flat damping characteristic over the frequency. The damping should, ideally, be completely flat over the full frequency range, except for in the resonance frequency range. In the resonance frequency range, the damping is to be reduced to compensate for the missing resonance frequency due to the placement of the hearing protector.

The ratio of the area of the sound inlet opening to the area of the sound outlet opening may be 8:1, or anything close to that.

In an example, the length of said second tube part is between 1 mm - 2mm, preferably substantially 1,5mm.

It was found that the length of the second tube part may have an impact on the damping characteristic. A second tube part length of between 1 mm - 2mm is beneficial for obtaining the desired damping.

In a further example, the filter comprises at least one O-ring, wherein each O-ring has a membrane attached thereto, wherein said at least one O-ring is provided in said first tube part.

Different types of membranes, permeable or non-permeable, exist that accomplish a particular damping. For example, an air permeable membrane can be realized by the use of an air-permeable porous material which is provided with micropores. By increasing the number of perforations and/or the diameter thereof, the damping of low frequencies by the membrane can be reduced. By providing perforations of substantially equal diameter, the low frequency damping can simple and reproducibly be stepwise influenced. Another example is to use an air tight membrane.

In an example of the present disclosure, the membrane is a foil, i.e. an air tight membrane that is transparent for moisture by means of absorption on one side and evaporation on the other side of the foil, whereas a mesh membrane is actually not air tight.

Typically, the filter comprises two O-rings placed one after the other in the first tube part. The first O-ring may have attached thereto a different membrane compared to the second O-ring, or may have attached thereto the same membrane, for example two foils.

In an example, the diameter of a passage through each O-ring is between 2mm - 4mm, preferably around 3mm.

The above still has the effect that the sound passage from the sound inlet opening to the sound outlet opening is reduced. The effective area of the sound inlet opening may be defined by the diameter of the passage through the O-ring. The second tube part may have a diameter smaller than that particular diameter of the passage through the O-ring.

In yet another example, the first tube part has a first axis of rotation symmetry, and wherein said second tube part has a second axis of rotation symmetry, wherein said first and said second axis of rotation symmetry coincide.

The above entails that the second tube part and the first tube part are in line with each other. Alternatively, the centre axis of the second tube part may be offset to the centre axis of the first tube part.

In a second aspect, there is provided a hearing protector placeable in the auditory duct of a person, said hearing protector comprising a sound damping filter in accordance with any of the examples as provided above.

The above-mentioned and other features and advantages of the invention will be best understood from the following description referring to the attached drawings. In the drawings, like reference numerals denote identical parts or parts performing an identical or comparable function or operation.
Figure 1 discloses a sound damping filter in accordance with the present disclosure.
Figure 2 discloses a Sound Pressure Level to frequency chart.

It is noted that the figures are only schematic representations of preferred embodiments of the invention. In the figures, the same or like parts have been indicated by corresponding reference numerals.

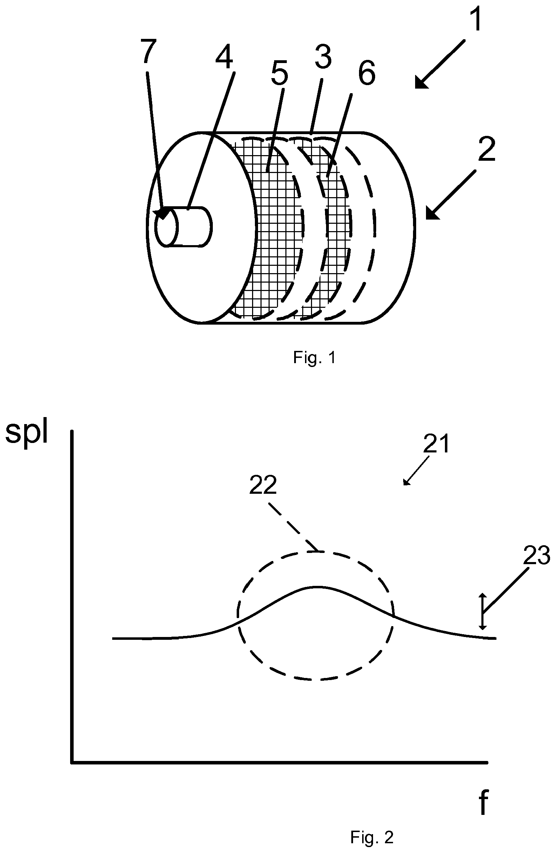

Figure 1 is an example of a sound damping filter 1 in accordance with the present disclosure. The sound damping filter 1 is to be placed in a hearing protector placeable in the auditory duct of a person. Such a hearing protector is arrange to substantially close of the auditory duct, such that the sound traverses only or mostly the sound damping filter inside the hearing protector.

The sound damping filter 1 comprises a sound damping canal, wherein the sound damping canal comprises a first tube part 3 connected to a second tube part 4, wherein the first tube part 3 provides for a sound inlet opening 2 and wherein the second tube part 4 provides for a sound outlet opening 7 such that a sound passage extends in said sound damping canal from the sound inlet opening 2 to said sound outlet opening 7.

Here, two membranes 5, 6 are provided in the first tube part 3 for providing a sound damping function. The membranes may be air-permeable or air tight. In accordance with the present disclosure, the membranes are used for providing a particular sound damping function, but they are not limited to a particular technique, material, elasticity, density, surface property, permeability, or anything alike.

The auditory duct of a person typically acts as a resonant cavity with one closed end and one open end, i.e. a ¼ wavelength resonator. Given the speed of sound in air, i.e. approximately 343 meters per second, standing frequency waves may develop inside the auditory duct. More specifically, the auditory duct may be exposed to a resonance frequency typically somewhere between 2.5 Khz - 3.5 Khz. This effect is a part of the natural perception of sound by the human auditory system.

The present disclosure deals with the issue that once a hearing protector is placed in the auditory duct, the characteristics of the auditory duct are changed. This results in the concept that the open ear standing resonant frequency will no longer develop in the auditory duct, as the auditory duct is no longer open at one side thereof. It is noted that the auditory duct may still be vulnerable for resonance frequencies, but the corresponding frequencies may be in a much higher frequency spectrum and therefore do not add to the natural sound perception.

The inventors have found that the hearing protector should attenuate the frequencies of between roughly 2.5 Khz - 3.5 Khz less to compensate for the loss of the oscillation frequencies. This would result in a concept in which the human auditory system perceives as if no hearing protector is present in the ear, and that the incoming/received sound waves are quite naturally spread over the frequency range, while at the same time the total exposure is significantly reduced.

This is accomplished by the use of a membrane in combination with the second tube part 4, i.e. a hollow protrusion part connected to the first tube part 3. The relatively small extension ensures the desired damping characteristic over the frequency range as indicated.

Preferably, the sound outlet opening 7, is round and has a diameter of approximately 0,6 mm. This has proven to provide for acceptable results. The sound inlet opening 2 is, preferably, also round and has, preferably, a diameter of approximately 5 mm.

The length of the second tube part 4, as well as the length of the first tube part 3, may also have an impact on the obtained damping characteristics. Preferably, the length of the second tube part is approximately 1,5 mm, and the length of the first tube part is approximately 4 mm.

It is noted that the first tube part 3, the second tube part 4, the O-rings 5, 6 may be of a material comprising any of polypropylene, styrene-ethylene-butylene-styrene polyurethanes, silicones, polyethylenes, fluoropolymers, and copolymers thereof.

Figure 2 discloses a Sound Pressure Level to frequency chart 21. Here, it is clear that a resonance frequency range 22 is developed in the auditory duct of a person, which is, typically, about 10 - 15 dB higher 23 compared to the nominal level.

The present disclosure is not limited to the embodiments as disclosed above, and can be modified and enhanced by those skilled in the art beyond the scope of the present disclosure as disclosed in the appended claims without having to apply inventive skills.

## Claims

1. A sound damping filter for a hearing protector placeable in the auditory duct of a person, said sound damping filter comprising a sound damping canal, wherein said sound damping canal comprises a first tube part connected to a second tube part, wherein said first tube part provides for a sound inlet opening and wherein said second tube part provides for a sound outlet opening such that a sound passage extends in said sound damping canal from said sound inlet opening to said sound outlet opening, wherein said first tube part comprises at least one membrane providing a sound damping function, and wherein said sound inlet opening is larger than said sound outlet opening, wherein
- said first tube part is formed such that said sound inlet opening has a diameter of between 4mm - 6mm, preferably substantially 5mm, and
- said second tube part is formed such that said sound outlet opening has a diameter of between 0,3mm - 1,0mm, preferably substantially 0,6mm
- a length of said second tube part is between 1 mm - 2mm, preferably substantially 1,5mm.

2. A sound damping filter in accordance with claim 1, wherein said filter comprises at least one O-ring, wherein each O-ring has a membrane attached thereto, wherein said at least one O-ring is provided in said first tube part, wherein said membrane is a foil.

3. A sound damping filter in accordance with claim 2, wherein said filter comprises two O-rings.

4. A sound damping filter in accordance with claim 3, wherein a diameter of a passage through each O-ring is between 2mm - 4mm, preferably around 3mm.

5. A sound damping filter in accordance with any of the previous claims, wherein said first tube part and said second tube part are shaped cylindrically.

6. A sound damping filter in accordance with any of the previous claims, wherein said first tube part has a first axis of rotation symmetry, and wherein said second tube part has a second axis of rotation symmetry, wherein said first and said second axis of rotation symmetry coincide.

7. A hearing protector placeable in the auditory duct of a person, said hearing protector comprising a sound damping filter in accordance with any of the previous claims.

## Patentansprüche

1. Schalldämpfungsfilter für einen in den Gehörgang einer Person platzierbaren Gehörschutz, wobei der Schalldämpfungsfilter einen Schalldämpfungskanal umfasst, wobei der Schalldämpfungskanal einen ersten Rohrteil umfasst, der mit einem zweiten Rohrteil verbunden ist, wobei der erste Rohrteil eine Schalleinlassöffnung bereitstellt und wobei der zweite Rohrteil eine Schallauslassöffnung bereitstellt, so dass sich ein Schalldurchgang in dem Schalldämpfungskanal von der Schalleinlassöffnung zu der Schallauslassöffnung erstreckt, wobei der erste Rohrteil mindestens eine Membran umfasst, die eine Schalldämpfungsfunktion bereitstellt, und wobei die Schalleinlassöffnung größer als die Schallauslassöffnung ist, wobei
- der erste Rohrteil derart ausgebildet ist, dass die Schalleinlassöffnung einen Durchmesser von zwischen 4 mm - 6 mm, vorzugsweise im Wesentlichen 5 mm, aufweist, und
- der zweite Rohrteil derart ausgebildet ist, dass die Schallauslassöffnung einen Durchmesser von zwischen 0,3 mm - 1,0 mm, vorzugsweise im Wesentlichen 0,6 mm, aufweist,
- eine Länge des zweiten Rohrteils zwischen 1 mm - 2 mm, vorzugsweise im Wesentlichen 1,5 mm beträgt.

2. Schalldämpfungsfilter nach Anspruch 1, wobei der Filter mindestens einen O-Ring umfasst, wobei jeder O-Ring eine daran angebrachte Membran aufweist, wobei der mindestens eine O-Ring in dem ersten Rohrteil bereitgestellt ist, wobei die Membran eine Folie ist.

3. Schalldämpfungsfilter nach Anspruch 2, wobei der Filter zwei O-Ringe umfasst.

4. Schalldämpfungsfilter nach Anspruch 3, wobei ein Durchmesser eines Durchgangs durch jeden O-Ring zwischen 2 mm - 4 mm, vorzugsweise etwa 3 mm beträgt.

5. Schalldämpfungsfilter nach einem der vorhergehenden Ansprüche, wobei der erste Rohrteil und der zweite Rohrteil zylindrisch geformt sind.

6. Schalldämpfungsfilter nach einem der vorhergehenden Ansprüche, wobei der erste Rohrteil eine erste Rotationssymmetrieachse aufweist, und wobei der zweite Rohrteil eine zweite Rotationssymmetrieachse aufweist, wobei die erste und die zweite Rotationssymmetrieachse deckungsgleich sind.

7. Gehörschutz, der in den Gehörgang einer Person platzierbar ist, wobei der Gehörschutz einen Schalldämpfungsfilter nach einem der vorhergehenden Ansprüche umfasst.

## Revendications

1. Filtre insonorisant pour un protecteur auditif pouvant être placé dans le conduit auditif d'une personne, ledit filtre insonorisant comprenant un canal insonorisant, dans lequel ledit canal insonorisant comprend une première partie de tube reliée à une deuxième partie de tube, où ladite première partie de tube fournit une ouverture d'entrée du son et où ladite deuxième partie de tube fournit une ouverture de sortie du son de sorte qu'un passage du son s'étende dans ledit canal insonorisant à partir de ladite ouverture d'entrée du son vers ladite ouverture de sortie du son, où ladite première partie de tube comprend au moins une membrane assurant une fonction d'insonorisation, et où ladite ouverture d'entrée du son est plus grande que ladite ouverture de sortie du son, où
- ladite première partie de tube est formée de sorte que ladite ouverture d'entrée du son ait un diamètre compris entre 4 mm et 6 mm, de préférence sensiblement de 5 mm, et
- ladite deuxième partie de tube est formée de sorte que ladite ouverture de sortie du son ait un diamètre compris entre 0,3 mm et 1,0 mm, de préférence sensiblement de 0,6 mm
- une longueur de ladite deuxième partie de tube est comprise entre 1 mm et 2 mm, de préférence sensiblement de 1,5 mm.

2. Filtre insonorisant selon la revendication 1, dans lequel ledit filtre comprend au moins un joint torique, dans lequel chaque joint torique a une membrane fixée à celui-ci, dans lequel ledit au moins un joint torique est prévu dans ladite première partie de tube, dans lequel ladite membrane est une feuille.

3. Filtre insonorisant selon la revendication 2, dans lequel ledit filtre comprend deux joints toriques.

4. Filtre insonorisant selon la revendication 3, dans lequel un diamètre d'un passage à travers chaque joint torique est compris entre 2 mm et 4 mm, de préférence d'environ 3 mm.

5. Filtre insonorisant selon l'une des revendications précédentes, dans lequel ladite première partie de tube et ladite deuxième partie de tube sont de forme cylindrique.

6. Filtre insonorisant selon l'une des revendications précédentes, dans lequel ladite première partie de tube a un premier axe de symétrie de rotation, et dans lequel ladite deuxième partie de tube a un deuxième axe de symétrie de rotation, dans lequel ledit premier et ledit deuxième axe de symétrie de rotation coïncident.

7. Protecteur auditif pouvant être placé dans le conduit auditif d'une personne, ledit protecteur auditif comprenant un filtre insonorisant selon l'une des revendications précédentes.
